**Europäisches Patentamt**

**European Patent Office**

**Office européen des brevets**

(19)

(11) Veröffentlichungsnummer: **0 060 553**
**B1**

(12) # EUROPÄISCHE PATENTSCHRIFT

(45) Veröffentlichungstag der Patentschrift:
**27.12.85**

(51) Int. Cl.⁴: **A 61 K 9/08,** A 61 K 31/70

(21) Anmeldenummer: **82102101.1**

(22) Anmeldetag: **16.03.82**

(54) Präparat zur Behandlung von Wunden der Hautoberfläche und Verfahren zur Herstellung des Präparats.

(30) Priorität: **17.03.81 HU 66381**

(43) Veröffentlichungstag der Anmeldung:
**22.09.82 Patentblatt 82/38**

(45) Bekanntmachung des Hinweises auf die Patenterteilung:
**27.12.85 Patentblatt 85/52**

(84) Benannte Vertragsstaaten:
**AT BE CH DE FR GB IT LI NL**

(56) Entgegenhaltungen:
**FR - A - 2 160 246**

**ROTE LISTE, 1961, Editio Cantor, Aulendorf/Wurtt.**

**Die Akte enthält technische Angaben, die nach dem Eingang der Anmeldung eingereicht wurden und die nicht in dieser Patentschrift enthalten sind.**

(73) Patentinhaber: **Humán Oltoanyagtermelö és Kutato Intézet, Táncsics M. u. 82, H-2100 Gödöllö (HU)**

(72) Erfinder: **Szijjártó, geb. Auber, Emilia, Dr., Szilágyi Erzsébet fasor 13, H-1024 Budapest (HU)**
Erfinder: **Ráskai, Magda, Dr., Darkai u. 20, H-1148 Budapest (HU)**

(74) Vertreter: **Lotterhos, Hans Walter, Dr.-Ing., Lichtensteinstrasse 3, D-6000 Frankfurt am Main 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Präparate zur Behandlung von Wunden der Epithelschicht der Haut sowie die Herstellung derartiger Präparate.

Es sind zahlreiche Verfahren zur Behandlung von Verletzungen der Epithelschicht der Haut — vor allem zur Behandlung von Brandwunden — bekannt. Die Begleitsymptome der Verletzungen, wie Hyperaesthesia, Schmerz, Hautrötung, in schwereren Fällen Blasenbildung und in sehr schweren Fällen Schädigung des Unterhautgewebes, Plasmagerinnung, Ödeme usw. hängen von der Schwere der Verletzungen ab.

Zur ersten Phase der Behandlung gehört im gegebenen Fall Kühlen der Hautoberfläche, Behebung des Schocks, Schmerzstillung, Wundsäuberung (Entfernung der Epithelstücke, Abscherung der Hautblasen, Abwaschen) und Prophylaxe gegen Wundinfektionen.

Da von den bekannten Wundbehandlungsmethoden die sogenannte »offene« Behandlung nur in einer speziellen, keimarmen Umgebung erwünscht ist (Sauerstoffzelt) wird gewöhnlich die »geschlossene« Verbandsmethode sehr viel häufiger angewandt.

Es gibt auch Übergangsmethoden zwischen den offenen und geschlossenen Behandlungen, z. B. die »Gerbbehandlung«, die die Exsudation herabsetzt, eine schmerzstillende Wirkung aufweist und die Proteolyseprodukte bindet (Tannin).

Eine spezielle Art der offenen Behandlung stellt die Anwendung der filmbildenden Gele dar, die mit dem geronnenen Plasma eine transparente impermeable Schicht auf der Wundoberfläche bilden. Die hierfür verwendeten Gelsorten sind im allgemeinen auf Kunststoffbasis (z. B. Polyvinylderivate; Vulnoplastin®, Aeroplast®) hergestellt. Ihre Anwendung ist bei der Behandlung von Brandwunden dritten Grades wegen der anaeroben Infektionsgefahr nicht erwünscht.

In der jüngsten Zeit sind bei Wundbehandlungen häufig folgende Arzneiformulierungen angewendet worden; Flüssigkeiten in Aerosol-Verpackungen, Pulver sowie Salben (Oxycort, Panthenol). Neben den oben angegebenen desinfizierenden Mitteln zur Wundsäuberung wurde eine 2%ige Mercurochromlösung erfolgreich angewandt, die noch durch Silbernitrat und Tannin ergänzt wurde.

Bei tiefen Brandwunden stellt die Anwendung der offenen Wundbehandlung ein Hindernis dar, und zwar weil sich die spontane Demarkation der Wunde verzögert herausbildet, und infolgedessen auch die Operation erst verzögert durchgeführt werden kann.

Bei der Heilung schwerer Brandwunden wurde neuerdings mit der Anwendung von lyophilisierter Schweinehaut als biologischem Verbandsmittel oder mit der Anwendung von synthetischem Wundabdeckungsmaterial (Pflastern, z. B. Epigard®) gute Resultate erzielt. Bei der geschlossenen Behandlungsform wird die Wunde vor Sekundärinfektionen durch ein steriles Verbandsmittel geschützt.

Nach der entsprechenden Säuberung werden die Wunden mit einem Chemotherapeutikum oder Antibiotikum, einem Streupuder oder einer Salbe behandelt.

Als Binde kann eine einfache, trockene Binde verwendet werden. Statt der Gaze wird oft ein spulennetzgewebtes Nylonfaser-Verbandmaterial verwendet. Oft werden die Verbandmaterialien imprägniert (Paraffin, Bienenwachs), hauptsächlich um dadurch das Festkleben des Verbands an der Wunde zu vermeiden. Eine spezielle Form der Hydrotherapie besteht darin, das Verbandmaterial in einem Bad von der Wunde abzuweichen (Artz, C. P.; Reiss, E.; Saunders, W. B.: Behandlung von Brandwunden (1957); Company János Gy., Novák, J.: Brandwunden (1967); Jakab, Lenz, Forgács: Intensive Krankenpflege, Bp. (1975); Lynch, J. B.; Lewis, S. R.: Symposium über die Behandlung von Brandwunden, Saint Louis, 1973; sowie J. of Traum, 13, 374—383 (1979)).

Der Nachteil der Verbandmethode besteht darin, daß das Verbinden der Wunde Schmerzen bereitet und daß das Verbandmaterial später entfernt werden muß. Das Verbandmaterial kann auch an der Wunde kleben oder die Wunde nicht vollständig verschließen, was zu Infektionen Anlaß geben kann. Bei einer sich stärker schließenden Wunddecke ist die Frage der Belüftung und die Entfernung des Sekrets ungelöst.

Die Erfindung betrifft Gerbstoff enthaltende Präparate, die vor allem zur Behandlung von Brandwunden, von durch Virus verursachten Herpes, zur Nachbehandlung von erfrorener Hautoberfläche, von Hautabschürfungen, von Hautverätzungen, zur Epithelförderung, zur Behandlung und Nachbehandlung des Keloids bzw. Hypertrophgewebes der Haut sowie zur Behandlung von Pyogeninfektionen der Haut geeignet sind und die in 100 ml $C_2—C_4$-Alkanollösung, vorzugsweise 70—100%igem Äthanol, 5—20 mg Gerbstoff, 5—15 mg Kohlenhydrat, 0,5—6 mg einer oder mehrerer Verbindung(en) von Anthocyantyp und/oder eine Verbindung von Flavonontyp und/oder Pectin, 0,5—6 mg Pflanzenwachs und/oder 0,01—0,1 mg ätherische Öle enthalten.

Die Erfindung betrifft auch die Herstellung dieser Präparate.

In diesen Präparaten können

als Gerbstoffe Brenzcatechin, Gallussäure, Tannin, Digallussäure, Pentadigalloyl-Glucose,
als Kohlenhydrat Glucose, Fructose, Rhamnose und/oder Xylose,
als Flavonon-Verbindung Quercetin sowie dessen Derivate,
als Verbindung von Anthocyan-Typ, Anthocyan als Wirkstoffkomponente, und/oder Cyanidin oder die Derivate dieser Verbindungen angewendet werden.

Bei der Herstellung des Präparates der Erfindung ist die Anwendung ätherischer Öle sehr wichtig. Zu diesem Zweck können 40—90% Geraniol, 4—15% Nerol, 10—50% Citronellol, Eugenol und/oder Linalool bzw. die entsprechenden Aldehyde verwendet werden.

Die angegebenen Komponenten sind in dem Präparat der Erfindung unerläßlich, weil sie die Desinfizierung der Wundoberfläche, die Abdeckung der Wunde und die Förderung der Epithelbildung — die den Wundschmerz lindert — sichern.

Es ist sehr vorteilhaft, die Wirksamkeit des Präparates der Erfindung durch weitere Zusatzstoffe, wie z. B. Vitamine, zu verbessern. Einige Beispiele hierfür sind die Vitamine: A, $B_1$, $B_6$, $B_{12}$, C, K und/oder P.

Als weitere Zusatzstoffe können auch anorganische Salze, z. B. Phosphate und/oder Chloride und/oder Spurenelemente in Spurendosis, wie Mangan-, Magnesium-, Calcium-, Cobalt-, Eisen-, Zinkionen, verwendet werden.

Pflanzenhormone, z. B. Auxin, Kinetin, und Enzyme mit oxydativer Wirkung, z. B. Peroxydase, Katalase, werden als weitere Zusatzstoffe zugesetzt.

Die einzelnen Komponenten können gemeinsam in Äthanol gelöst werden, wenn es sich um Äthanol-lösliche Materialien handelt. Es ist zweckmäßig, die einzelnen Zusatzstoffe zuerst ineinander oder in wenig Wasser aufzulösen und erst danach in das Präparat umzulösen. Gegebenenfalls kann ein Lösungsvermittler angewendet werden (z. B. Äther).

Es hat sich als zweckmäßig erwiesen, wenn die Vitamine A und K in ätherischen Ölen gelöst dem System zugegeben werden. Das Vitamin C wird in konzentrierter wäßriger Lösung zugesetzt.

Das Lösen der einzelnen Komponenten erfolgt vorzugsweise bei 0—30°C, also bei niedrigerer Temperatur als Raumtemperatur. Aus den einzelnen Komponenten werden zweckmäßig Stammlösungen — möglichst in Äthanol aber auch in Wasser — hergestellt, mit denen dann bei der Herstellung der Endkomposition die genaue Dosierung vorgenommen wird. Die Stammlösungen sollen bis zum Verbrauch in einem dunklen, kühlen Raum aufbewahrt werden.

Die Äthanollösung, die die Hauptkomponenten enthält, ist — wie gefunden wurde — sehr stabil und kann lange Zeit bei Raumtemperatur gelagert werden, ist aber vor Sonnenschein zu schützen.

Das Präparat der Erfindung wird auf der Wundoberfläche, nachdem diese gereinigt und/oder eventuell mit Wasser gekühlt, z. B. bei Verätzungen gewaschen und neutralisiert worden ist, sorgfältig verteilt und in einer dünnen Schicht aufgetragen.

Es ist zweckmäßig, das Präparat durch Einpinseln, Anfechten oder Spritzen auf die Haut- oder Wundoberfläche aufzubringen.

Diese Behandlung soll so lange wiederholt werden, bis das Schmerzgefühl nachläßt. Sie kann nötigenfalls mehrmals wiederholt werden. Die Wundoberfläche soll während der Behandlung vor Wasser und Seife geschützt werden.

Während der Behandlung entsteht eine dünne Filmschicht mit Atmungsöffnungen (Vakuolen) für die Wundoberfläche, die die Wunde vor Infektion schützt, während die Wirkstoffe des Erzeugnisses ihre epithelfördernde, desinfizierende bzw. schmerzstillende Wirkung zur Geltung bringen.

Die einzelnen Komponenten können unmittelbar als solche oder als Pflanzenextrakte dem Präparat zugegeben werden.

Die Erfindung soll durch die folgenden Beispiele näher erläutert werden. Hieraus sind jedoch keine Beschränkungen herzuleiten.

Beispiel 1

Es werden die folgenden Basislösungen vorbereitet:

Lösung I
1000 ml 96% äthanolische (PhHgVI)* Tinktur, die
- 10 g Gallussäure,
- 5 g Brenzcatechin,
- 0,5 mg Anthocyan,
- 0,2 mg Auxin und
- 0,15 mg Kinetin
enthält.

Lösung II
- 15 g Glucose (PhPgVi),
- 3 mg Vitamin C (Acidum Ascorbinicum) (PhHgVI),
- 20 mg Vitamin $B_1$ (Aneurinum Hydrochloricum) (PhHgVI),
- 20 mg Vitamin $B_6$ (Pyridoxinum Hydrochioricum) (PhHgVI),
- 10 mg Vitamin $B_{12}$ (Cyanocobalaminum) (PhHgVI),
- ad 1000 ml 25% Äthylalkohol.

3

Lösung III

| | |
|---|---|
| 4 g | Cera Alba, |
| 25 mg | Geraniol, |
| 7 mg | Nerol, |
| 15 mg | Citronellol, |
| 3 mg | Eugenol, |
| 1 mg | Carotin, |
| ad   1000 ml | Äthyläther (PhHgVl). |

*) PhHg = Pharmacopola Hungarica.


Die Basislösungen werden wie folgt gemischt:

10 ml Lösung I,
10 ml Lösung II,
10 ml Lösung III,
ad   1000 ml 96% Äthylalkohol (PhHgVl).

Die so erhaltene Lösung wird durch eine alkoholresistente Membrane steril filtriert und als Spray in laschen gefüllt.


Beispiel 2

Es werden die folgenden Basislösungen vorbereitet:

Lösung I
1000 ml 96% äthanolische (PhHgVl) Tinktur, die

| | |
|---|---|
| 5 g | Digallussäure, |
| 0,8 g | Anthocyan, |
| 2,6 g | Cyanidin, |
| 15 g | Acidum Tannicum (PhHgVl), |
| 0,15 mg | Auxin und |
| 0,1 mg | Kinetin |

enthält.


Lösung II

| | |
|---|---|
| 10 g | Glucose (PhHgVl), |
| 5 g | Rhamnose (PhHgVl), |
| 20 mg | Vitamin C (Acidum Ascorbinicum) (PhHgVl), |
| 3 mg | Vitamin P (Rutin), |
| 0,2 mg | Peroxydase, |
| ad   1000 ml | destilliertem Wasser. |

Lösung III

| | |
|---|---|
| 2 mg | Vitamin A (Axerolphthalum) (PhHgVl), |
| 70 mg | Geraniol, |
| 4 mg | Nerol, |
| 20 mg | Citronellol, |
| 3 mg | Eugenol, |
| 3 mg | Linalool, |
| 2 g | Cera Flava, |
| 4 g | Cera Alba, |
| ad   1000 ml | Äthyläther (Äther ad narcosum) (PhHgVl). |

Lösung IV

| | |
|---|---|
| 20 mg | Vitamin B$_1$ (Aneurinum Hydrochloricum) (PhHgVl), |
| 15 mg | Vitamin B$_6$ (Pyridoxinum Hydrochloricum) (PhHgVl), |
| 5 mg | Vitamin B$_{12}$ (Cyanocobalaminum) (PhHgVl), ad1000 ml25% Äthylalkohol (PhHgVl). |

Die Basislösungen werden wie folgt gemischt:

    10 ml Lösung I,
    10 ml Lösung II,
    10 ml Lösung III,
    10 ml Lösung IV,
ad    1000 ml 96% Äthylalkohol (PhHgVI).

Die so erhaltene Lösung wird durch eine alkoholresistente Membrane steril filtriert und als Spray in Flaschen gefüllt.


## Beispiel 3

Die folgenden Ausgangsmaterialien:

| 10 mg | Gallussäure, |
| 5 mg | Brenzcatechin, |
| 15 mg | Glucose, |
| 0,5 mg | Anthocyan, |
| 4 mg | Cera Alba (in Äther gelöst), |
| 0,05 mg | in Äther gelöste ätherische Öle folgender Zusammensetzung: |
| | 50% Geraniol, |
| | 15% Nerol, |
| | 30% Citronellol, |
| | 5% Eugenol, |
| 0,003 mg | Vitamin C und |
| 0,05 mg | Vitamine B, $B_1$, $B_6$, $B_{12}$ (in gleicher Menge) |

werden in sterilem 96%igem Äthanol gelöst, so daß man 100 ml Lösung erhält. Die erhaltene Lösung wird steril filtriert und als Spray in Flaschen gefüllt.


## Beispiel 4

Die folgenden Ausgangsmaterialien:

| 15 mg | Tannin, |
| 5 mg | Digallussäure, |
| 0,8 mg | Anthocyan, |
| 2,6 mg | Cyanidin, |
| 10 mg | Glucose, |
| 5 mg | Rhamnose, |
| 6 mg | Pflanzenwachs |
| 0,1 mg | in Äther gelöste ätherische Öle folgender Zusammensetzung: |
| | 70% Geraniol, |
| | 4% Nerol, |
| | 10% Citronellol, |
| | 3% Eugenol, |
| | 3% Linalool, |
| 0,02 mg | Vitamin C, |
| 0,04 mg | Vitamin B, |
| 0,02 mg | Vitamin A (das in die Ölphase eingebracht wird) und |
| 0,003 mg | Vitamin P |

werden in sterilem 96%igem Äthanol zu 100 ml Lösung gelöst.


## Beispiel 5

Mit 96%igem Äthanol wird eine Lösung aus folgenden Ingredienten hergestellt:

| 5,25 g | Digalloyl-Glucose, |
| 3,00 g | Penta-Digalloyl-Glucose, |
| 0,75 g | Gallusäure, |

| | |
|---|---|
| 6,00 g | Catechin-Gerbsäure, |
| 0,90 g | Quercetin, |
| 0,75 g | Campher, |
| 0,60 g | Apigenin, |
| 0,75 g | Delphinin, |
| 0,75 g | Cyanin, |
| 3,75 g | Glucose, |
| 3,00 g | Fructose, |
| 0,30 g | Ribose, |
| 0,15 g | Xylose, |
| 12,80 g | Ultra-Amino-Pektin, |
| 3,75 g | Bienenwachs, |
| 3,75 g | Carnauba-Wachs, |
| 0,0045 g | Geraniol, |
| 0,0015 g | Nerol, |
| 0,003 g | Citronellol, |
| 0,003 g | Citral, |
| 0,0003 g | Eisen(III)chlorid, |
| 0,0002 g | Mangan(II)chlorid, |
| 0,0001 g | Cobalt(II)chlorid, |
| 0,0001 g | Magnesium(II)chlorid. |

Die erhaltene Lösung wird mit Äthanol auf 1000 g ergänzt, danach mit 96%igem Äthanol auf das 100fache verdünnt, durch eine Äthanol-resistente Membrane filtriert und steril als Spray abgefüllt. Die einzelnen Bestandteile können auch aus pflanzlichen Extrakten in Gruppen gewonnen und alsdann vermischt werden.

## Charakteristische Merkmale der Präparate der Erfindung und deren Bestimmung

### A. Trockensubstanzgehalt

Prüfungsmethode gemäß Seite 110, Punkt 2.2 der PhHgVI Band I.
Der Trockensubstanzgehalt der Lösung soll min. 0,03% betragen.

### B. Zuckergehalt

Zuckerbestimmung, kolorimetrisch (Anthron). (Literatur: R. L. Whistler, W. L. Wolfram: Methods in Carbohydrate Chemistry. I., Academic Press, New York, 1962).
Der Zuckergehalt der Lösung soll etwa 40—50% des Trockensubstanzgehaltes betragen.

### C. Gerbstoffgehalt

Gerbstoffprüfung, kolorimetrisch nach Mattil und Filer (Literatur: Kakác-Vejdelek: Handbuch der Kolorimetrie, II. V. G. Fischer Verlag, Jena, 1963).
Der Gerbstoffgehalt der Lösung soll etwa 20% des Trockensubstanzgehaltes betragen.

### D. Qualitative Anforderungen an die Lösung therapeutischer Konzentration

Die Lösung soll hellgelb-rosarot, durchsichtig und kristallklar sein und darf keine Sedimente enthalten.

1. Prüfung der Farbstoffe
   5 ml der Lösung werden gemäß Punkt 1.1 auf Seite 102 des Bandes I von PhHgVI geprüft.
   Die Farbe der Lösung soll zwischen den Farben 3—5 der Farbmeßlösungen liegen.
2. Acidität-, Alkalitätsprüfung
   Der pH-Wert der mit frisch ausgekochtem und abgekühltem destilliertem Wasser in einem Verhältnis von 1 : 9 verdünnten Lösung soll — mit einem elektrischen pH-Meßgerät gemessen — zwischen 5,00 + 6,00 liegen.
3. Dichte
   Der bei 20° C gemessene Wert soll zwischen 0,804 + 0,806 — nach PhHgVI — liegen.

**0 060 553**

### E. Prüfung auf akute Toxizität

Zur einmaligen Behandlung der max. zu 50% verbrannten Körperfläche eines Erwachsenen von 60 kg werden max. 200 ml Lösung angewendet. Bei einer dreimaligen Behandlung täglich können einem Erwachsenen mit schweren Brandwunden 600 ml Lösung appliziert werden. Der Trockensubstanzgehalt dieser Lösung beträgt insgesamt 200 mg und dessen auf das Körpergewicht umgerechnete Menge 200 : 60 = 3,0 mg Trockensubstanz/kg.

Bei den Toxizitätsprüfungen, die mit Mäusen und Kaninchen durchgeführt wurden, wurden hingegen folgende Dosen angewendet:

Die Dosis wurde auf Grund des Trockensubstanzgehaltes bestimmt.

Bei den Prüfungen wurde das Präparat eingedampft und danach mit einer 1%igen wäßrigen Natriumhydrocarbonatlösung — pH-Wert = 8,2 — wieder in Lösung gebracht und steril filtriert.

Die Prüfungsergebnisse sind in der nachfolgenden Tabelle zuammengefaßt:

| | Anzahl der Tiere | Art der Impfung | Dosis mg/kg | Menschliche Dosis/kg × Faktor | Beobachtungs- dauer | Reaktion |
|---|---|---|---|---|---|---|
| Maus | 12 | i.p. | 38,5 | 13 × | 2 Monate | 0 |
| Maus | 12 | i.p. | 77,0 | 26 × | 2 Monate 2 Monate | 2,3 Tage |
| Kaninchen | 12 | i.v. | 8,0 | 2,7 × | 2 Wochen— 2 Monate | 0 |
| Kaninchen | 5 | i.v. | 20,0 | 6,7 × | 2 Wochen— 2 Monate | 0 |
| Kaninchen | 5 | i.v. | 100 | 33 × | 2 Wochen— 2 Monate | 0 |

Das Körpergewicht der für die Prüfung benutzten Tiere betrug:

Maus        20 g
Kaninchen   2,5—3,5 kg.

Auf Grund dieser Versuchsergebnisse ist festzustellen, daß das 30fache der Dosis, die maximal an einem Tag auf die menschliche Haut aufgebracht wird, bei Kaninchen nicht toxisch ist.

### F. Behandlung von durch glühenden Stahl erzeugten Brandwunden

Das Fellhaar des Versuchskaninchens wurde abrasiert und danach auf dem Rücken des narkotisierten Kaninchens durch ein zum Glühen erhitztes Stahlplättchen von 6 cm² Größe eine Verbrennung dritten Grades erzeugt. Verbrennungsdauer: 2—5 bzw. 2 × 5 sec.

Nach der Verbrennung wurde die Wunde mit kaltem Wasser gekühlt (gewaschen) und nach 15—20 Minuten die Behandlung mit dem Präparat der Erfindung durch zweimaliges Spritzen durchgeführt. Die Behandlung wurde nach 4—5 Stunden wiederholt.

Weitere Behandlungen erfolgten täglich zweimal bis zur Abtrennung des Schorfrandes, danach erfolgte die Behandlung täglich einmal, später an jedem zweiten Tage. Die sich abtrennenden Schorfteile wurden mit der Schere entfernt. Kontrollbehandlungen wurden mit Vaseline, Oxycort, Alkohol, Panthenol, Mercurochrom und Silbernitrat durchgeführt.

Gesamtzahl der verbrannten Oberflächen:
121, die mit dem Präparat der Erfindung behandelt wurden,
110, die den Kontrollbehandlungen unterworfen wurden.

7

Im Laufe der Behandlungen mit dem Präparat der Erfindung wurde festgestellt, daß auf der verbrannten Oberfläche und in ihrer Umgebung weder ein Ödem noch eine Entzündung auftrat. Am 2.—3. Tag bildete sich auf der verbrannten Oberfläche ein harter Schorf, der sich zwischen dem 8. und 10. Tag abzutrennen begann. Darunter war eine gesunde Epithelschicht zu beobachten. Die volle Abtrennung des Schorfes konnte (je nach Schwere der Verbrennung) zwischen dem 15. und 25. Tag nach der Verbrennung beobachtet werden. In allen Fällen bildete sich unter dem abgetrennten Schorf eine gesunde Epithelschicht. Auch Haarwuchs konnte beobachtet werden.

An den Kontrolloberflächen waren dagegen kurz nach der Verbrennung Ödeme und Entzündungen zu beobachten und die Abtrennung des Schorfs erfolgte 6—8 Tage später. Unter dem abgetrennten Schorf war oft eine Fläche mit Epithelmangel zu beobachten, was die vollständige Heilung weitere 8—10 Tage verzögerte. Auch der Haarwuchs setzte erst später ein. In einzelnen Fällen war überhaupt kein Haarwuchs zu beobachten.

Die Heilungsdauer ist in der folgenden Tabelle angegeben:

| Anzahl der verbrannten Oberflächen | Behandlungsmethode | Heilungsdauer Tage |
|---|---|---|
| 121 | Präparat der Erfindung | 15—30 |
| 24 | 0,5% Silbernitrat | 15—26 |
| 25 | 2% Mercurochrom | 25—32 |
| 24*) | Oxycort | 28—60 |
| 5 | Panthenol | wegen Verenden von Tieren nicht auswertbar |
| 32 | 96% Äthylalkohol | 23—40 |
| 2 | Borvaseline | 36—40 |

*) = Wegen Verenden der Tiere waren nur 15 Fälle auswertbar.

Die Heilungsdauer stand in engem Verhältnis zur Verbrennungsdauer.

### G. Mikroskopische Pürfung der auf der Hautoberfläche entstehenden Filmschicht

Die natürlichen Farbstoffe, die in der Lösung therapeutischer Konzentration enthalten sind, reichen nicht aus, um die Vakuolen bei der mikroskopischen Prüfung sichtbar zu machen. Deshalb wird eine Färbung mit Säure-Fuchsin angewendet. In etwa 20 ml Lösung wird 0,05 g kristalline Farbe gelöst und die Lösung mit analytischem Filterpapier und anschließend mit einem Membranefilter filtriert. Die steril filtrierte Lösung wird danach mit einem Kapillarrohr auf einen entsprechend entfetteten Objektträger aufgetropft und eingedampft. Nach mehrmaliger Wiederholung dieses Vorgangs wird der Objektträger unter einem Mikroskop bei 100facher Vergrößerung untersucht. Die Vakuolen sind in dieser Schicht gleichmäßig verteilt.

### H. Prüfung der bakteriostatischen Wirkung

Der Trockensubstanzrückstand des eingedampften Präparats wurde in einer 1%igen wäßrigen Natriumhydrogencarbonatlösung gelöst und steril filtriert.

Von der erhaltenen alkoholfreien Lösung wurde eine Menge, die einer Konzentration von 5 mg/ml entsprach, einem Bouillonnährboden zugegeben, der mit verschiedenen Bakterienstämmen beimpft wurde, die in einer 24stündigen, flüssigen, 37°C-Kultur lebten. Das Wachstum der Bakterien wurde nach 24 Stunden notiert.

Die Resultate sind in der nachfolgenden Tabelle zusammengefaßt:

| Stamm | Wachstum | Kontrolle | Kultur |
|---|---|---|---|
| Staphylococcus aureus 112 001 | kein | normal | Anwuchs |
| Staphylococcus aureus 112 002 | kein | normal | Anwuchs |
| Staphylococcus aureus 306 | kein | normal | Anwuchs |
| Staphylococcus aureus 211 | kein | normal | Anwuchs |
| Staphylococcus aureus 223 | kein | normal | Anwuchs |
| Staphylococcus aureus Wood 46 | kein | normal | Anwuchs |
| Bacillus subtiles | normal | normal | Anwuchs |
| Proteus vulgaris 61 370 | normal | normal | Anwuchs |
| Pyocyaneus aeruginosa 170 014 | normal | normal | Anwuchs |

Bei der Untersuchung der alkoholischen Lösung wurden dieselben Stämme und dieselbe Methode angewendet, die oben angewendet ist, wobei etwa die gleichen Ergebnisse erhalten wurden.

### J. Kontrolle des Heilungsvorgangs durch Reaktionen auf mechanische Irritationen

Auf die mechanische Irritation (Druck) der verbrannten Oberfläche reagierten die Tiere mit Zusammenzucken der Haut und Verhaltensänderung eventuell mit Schmerzlauten. Auch ihr allgemeines Verhalten (Bewegungs-Nahrungsanspruch) kann zur Bewertung herangezogen werden. Bei der Berührung der mechanisch verbrannten Hautoberfläche, die mit dem Präparat der Erfindung behandelt worden war, blieben die Tiere ruhig. Die mit den Kontrollmitteln behandelten Tiere reagierten dagegen auf die Berührung in der oben angegebenen Weise, wobei auch Schmerzlaute wahrgenommen wurden.

Wurden an einem Tier mehrere verbrannte Flächen mit den Kontrollmitteln behandelt, nahmen die Tiere keine Nahrung zu sich und verloren auch an Körpergewicht. An einzelnen Tieren wurden auf der enthaarten Haut an 12 verschiedenen Stellen 6 cm² große Brandwunden verursacht, die mit dem Präparat der Erfindung behandelt wurden. Diese Tiere nahmen unmittelbar nach der Behandlung Nahrung zu sich und ihr Körpergewicht entwickelte sich normal.

Diese Untersuchungen wurden an 2,5—3,5 kg schweren Kaninchen durchgeführt.

### K. Behandlung von durch Verbrühen erzeugten Brandwunden

Der abrasierte Rücken je eines Kaninchens wurde mit 100°C heißem Wasser in Berührung gebracht. Diese Hautoberfläche entspricht etwa 30% der gesamten Hautoberfläche des Tieres. Das mit dem Präparat der Erfindung behandelte Tier wurde innerhalb von 4 Wochen vollkommen geheilt und zeigte normalen Haarwuchs an der behandelten Stelle. An dem Tier, das zur Kontrolle mit 96%igem Alkohol behandelt wurde, ist nach einer Behandlung von 3,5 Monaten eine Epithelmangelfläche von der Größe der Fläche einer Kinderhand zu beobachten.

### L. Kontrolle des Heilungsvorgangs durch Gewichtszunahme

Die therapeutische Wirkung läßt sich gut durch die Kurve des Körpergewichts demonstrieren, da während der Verbrennungskrankheit die Nahrungsaufnahme von dem negativen Stickstoffgleichgewicht und dem Allgemeinzustand des Tieres — der eng mit dem Schmerzgefühl zusammenhängt — bestimmt wird. Deshalb wurde das Körpergewicht von Tieren, die an 8—10 Stellen mechanisch mit einem 6 cm² großen Stahlplättchen verbrannt worden waren, während der Heilungsdauer registriert.

Das Körpergewicht des Kaninchens, das ausschließlich mit dem Präparat der Erfindung behandelt worden war, nahm nach der Verbrennung allmählich zu. Die Gewichtszunahme — verglichen mit dem ursprünglichen Gewicht — betrug insgesamt 950 g.

Das ausschließlich mit Silbernitrat behandelte Kaninchen verlor in 13 Tagen 200 g an Körpergewicht. Es erreichte sein ursprüngliches Gewicht erst am 20. Tag und nahm bis zum 36. Tag nur 200 g zu.

9

Das ausschließlich mit Mercurochrom behandelte Kaninchen verlor in 27 Tagen 600 g an Körpergewicht. Danach nahm sein Körpergewicht zu, aber am 36. Tag nach der Verbrennung lag sein Gewicht noch immer 350 g unterhalb seinem ursprünglichen Gewicht.

Das mit dem Präparat der Erfindung und mit Silbernitrat parallel behandelte Kaninchen verlor bis zum 3. Tag nach der Verbrennung 200 g an Körpergewicht und gewann sein ursprüngliches Körpergewicht erst am 9. Tag zurück. Am 36. Tag nach der Verbrennung betrug sein Gewicht 650 g mehr als sein ursprüngliches Körpergewicht.

Das mit dem Präparat der Erfindung und mit Äthylalkohol parallel behandelte Kaninchen verlor bis zum 3. Tag nach der Verbrennung 100 g an Körpergewicht. Sein ursprüngliches Körpergewicht hatte es am 13. Tag wieder erreicht. Am 36. Tag nach der Verbrennung betrug sein Gewicht 600 g mehr als sein ursprüngliches Körpergewicht.

**Patentansprüche für die Vertragsstaaten: BE, CH, DE, FR, GB, IT, LI, NL**

1. Gerbstoff enthaltendes Präparat zur Behandlung von Wunden der Hautoberfläche, dadurch gekennzeichnet, daß es in 100 ml $C_2 - C_4$-Alkanollösung

| | |
|---|---|
| 5—20 mg | Gerbstoff, |
| 5—15 mg | Kohlenstoff, |
| 0,5—6 mg | einer oder mehrerer Verbindung(en) vom Anthocyantyp und/oder einer Verbindung vom Flavonontyp und/oder Pectin, |
| 0,5—6 mg | Pflanzenwuchs und |
| 0,01—0,1 mg | ätherische Öle |

enthält.

2. Präparat nach Anspruch 1, dadurch gekennzeichnet, daß es als zusätzliche Komponenten

| | |
|---|---|
| 5—6 mg | Vitamine, Spurenelemente, Pflanzenhormone, Katalase, Enzyme mit oxydativer Wirkung und/oder anorganische Salze |

enthält.

3. Präparat nach Anspruch 1 oder 2, dadurch gekennzeichnet, daß es

als Gerbstoff Brenzcatechin, Tannin, Gallussäure, Digallussäure und/oder Pentadigalloyl-Glucose,
als Kohlenhydrat Glucose, Fructose, Rhamnose und/oder Xylose,
als Verbindung vom Anthocyantyp Anthocyan und/oder Cyanidin,
als Verbindung vom Flavonontyp Quercetin,
als ätherische Öle 40—90% Geraniol, 4—15% Nerol, 10—50% Citronellol, Eugenol, Linalool bzw. die entsprechenden Aldehyde,
als Vitamine die Vitamine A, $B_1$, $B_6$, $B_{12}$, C, K und/oder P,
als Spurenelemente Mangan, Magnesium, Calcium, Cobalt, Eisen und/oder Zink,
als Pflanzenhormone Auxin, Kinetin in ng-Mengen,
als Enzyme mit oxydativer Wirkung Peroxydase und/oder Katalase und
als anorganische Salze Phosphate und/oder Chloride

enthält.

4. Präparat nach Anspruch 1, 2 oder 3, dadurch gekennzeichnet, daß es als Lösungsmittel 70—100%iges Äthanol enthält.

5. Verfahren zur Herstellung von Gerbstoff enthaltenden Präparaten zur Behandlung der Hautoberfläche, dadurch gekennzeichnet, daß man

| | |
|---|---|
| 5—20 mg | Gerbstoff, |
| 5—15 mg | Kohlenhydrat, |
| 0,5—6 mg | einer oder mehrerer Verbindungen vom Anthocyantyp und/oder einer Verbindung vom Flavonontyp und/oder Pectin, |
| 0,5—6 mg | Pflanzenwachs und |
| 0,01—0,1 mg | ätherische Öle |

auf einmal oder verteilt in $C_2 - C_4$-Alkanol zu 100 ml Lösung löst.

6. Verfahren nach Anspruch 5, dadurch gekennzeichnet, daß man

| | |
|---|---|
| 5—6 mg | Vitamine, Spurenelemente, Pflanzenhormone, Katalase, Enzyme mit oxydativer Wirkung und/oder anorganische Salze |

als zusätzliche Komponenten verwendet.

7. Verfahren nach Anspruch 5 oder 6, dadurch gekennzeichnet, daß man

als Gerbstoff Brenzcatechin, Tannin, Gallussäure, Digallussäure und/oder Pentadigalloyl-Glucose,
als Kohlenhydrat Glucose, Fructose, Rhamnose und/oder Xylose,
als Verbindung vom Anthocyantyp Anthocyan und/oder Cyanidin,
als Verbindung vom Flavonontyp Quercetin,
als ätherische Öle 40—90% Geraniol, 4—15% Nerol, 10—50% Citronellol, Eugenol, Linalool bzw. die entsprechenden Aldehyde,
als Vitamine die Vitamine A, $B_1$, $B_6$, $B_{12}$, C, K und/oder P,
als Spurenelemente Mangan, Magnesium, Calcium, Cobalt, Eisen und/oder Zink,
als Pflanzenhormone Auxin, Kinetin in ng-Mengen,
als Enzyme mit oxydativer Wirkung Peroxydase und/oder Katalase und
als anorganische Salze Phosphate und/oder Chloride

verwendet.
8. Verfahren nach Anspruch 5, 6 oder 7, dadurch gekennzeichnet, daß man als Lösungsmittel 70—100%iges Äthanol verwendet.

**Patentansprüche für den Vertragsstaat: AT**

1. Verfahren zur Herstellung von Gerbstoff enthaltenden Präparaten zur Behandlung der Hautoberfläche, dadurch gekennzeichnet, daß man

5—20 mg    Gerbstoff,
5—15 mg    Kohlenhydrat,
0,5—6 mg   einer oder mehrerer Verbindungen vom Anthocyantyp und/oder einer Verbindung vom Flavonontyp und/oder Pectin,
0,5—6 mg   Pflanzenwuchs und
0,01—0,1 mg ätherische Öle

auf einmal oder verteilt in $C_2$—$C_4$-Alkanol zu 100 ml Lösung löst.
2. Verfahren nach Anspruch 1, dadurch gekennzeichnet, daß man

5—6 mg     Vitamine, Spurenelemente, Pflanzenhormone, Katalase, Enzyme mit oxydativer Wirkung und/oder anorganische Salze

als zusätzliche Komponenten verwendet.
3. Verfahren nach Anspruch 1 oder 2, dadurch gekennzeichnet, daß man

als Gerbstoff Brenzcatechin, Tannin, Gallussäure, Digallussäure und/oder Pentadigalloyl-Glucose,
als Kohlenhydrat Glucose, Fructose, Rhamnose und/oder Xylose,
als Verbindung vom Anthocyantyp Anthocyan und/oder Cyanidin,
als Verbindung vom Flavonontyp Quercetin,
als ätherische Öle 40—90% Geraniol, 5—15% Nerol, 10—50% Cintronellol, Eugenol, Linalool bzw. die entsprechenden Aldehyde,
als Vitamine die Vitamine A, $B_1$, $B_6$, $B_{12}$, C, K und/oder P,
als Spurenelemente Mangan, Magnesium, Calcium, Cobalt, Eisen und/oder Zink,
als Pflanzenhormone Auxin, Kinetin in ng-Mengen,
als Enzyme mit oxydativer Wirkung Peroxydase und/oder Katalase und
als anorganische Salze Phosphate und/oder Chloride

verwendet.
4. Verfahren nach Anspruch 1, 2 oder 3, dadurch gekennzeichnet, daß man als Lösungsmittel 70—100%iges Äthanol verwendet.

**Claims for the contracting States: BE, CH/LI, DE, FR, GB, IT, NL**

1. Product containing a tanning agent for treating wounds on the skin surface, characterized in that it contains in 100 ml of $C_2$—$C_4$ alkanol solution

5 to 20 mg    of tanning agent,
5 to 15 mg    of carbohydrate,

0.5 to 6 mg    of one or more compounds of the anthocyan type and/or a compound of the flavonone type and/or pectin,

0.5 to 6 mg    of vegetable wax and

0.01 to 0.1 mg of ethereal oils.

2. Product according to claim 1, characterized in that it contains as additional components

5 to 6 mg    of vitamins, trace elements, vegetable hormones, catalase, enzymes with an oxidative action and/or inorganic salts.

3. Product according to claims 1 or 2, characterized in that it contains

as the tanning agent pyrocatechol, tannin, gallic acid, digallic acid and/or pentadigalloyl-glucose,

as the carbohydrate glucose, fructose, rhamnose and/or xylose,

as a compound of the anthocyan type anthocyan and/or cyanidine,

as a compound of the flavonone type quercetin,

as essential oils 40 to 90% of geraniol, 4 to 15% of nerol, 10 to 50% of citronellol, eugenol, linalool or the corresponding aldehydes,

as vitamins A, $B_1$, $B_6$, $B_{12}$, C, K and/or P,

as trace elements manganese, magnesium, calcium, cobalt, iron and/or zinc,

as vegetable hormones auxin and kinetin in ng quantities,

as enzymes with an oxidative action peroxidase and/or catalase and

as inorganic salts phosphates and/or chlorides.

4. Product according to claims 1, 2 or 3, characterized in that it contains as the solvent 70 to 100% ethanol.

5. Process for the preparation of products containing a tanning agent for treating the skin surface, characterized in that

5 to 20 mg    of tanning agent,

5 to 15 mg    of carbohydrate,

0.5 to 6 mg    of one or more compounds of the anthocyan type and/or a compound of the flavonone type and/or pectin,

0.5 to 6 mg    of vegetable wax and

0.01 to 0.1 mg of ethereal oils,

are dissolved all at once or distributed in $C_2 - C_4$ alkanol to give 100 ml of solution.

6. Process according to claim 5, characterized in that

5 to 6 mg    of vitamins, trace elements, vegetable hormones, catalase, enzymes with an oxidative action and/or inorganic salts

are used as additional components.

7. Process according to claims 5 or 6, characterized in that

as the tanning agent pyrocatechol, tannin, gallic acid, digallic acid and/or pentadigalloyl-glucose,

as the carbohydrate glucose, fructose, rhamnose and/or xylose,

as a compound of the anthocyan type anthocyan and/or cyanidine,

as a compound of the flavonone type quercetin,

as essential oils 40 to 90% of geraniol, 4 to 15% of nerol, 10 to 50% of citronellol, eugenol, linalool or the corresponding aldehydes,

as vitamins, vitamins A, $B_1$, $B_6$, $B_{12}$, C, K and/or P,

as trace elements manganese, magnesium, calcium, cobalt, iron and/or zinc,

as vegetable hormones auxin and kinetin in ng quantities,

as enzymes with an oxidative action peroxidase and/or catalase and

as inorganic salts phosphates and/or chlorides are used.

8. Process according to claims 5, 6 or 7, characterized in that 70 to 100% ethanol is used as the solvent.

**0 060 553**

## Claims for the contracting state: AT

1. Process for the preparation of products containing a tanning agent for treating the skin surface, characterized in that

| | |
|---|---|
| 5 to 20 mg | of tanning agent, |
| 5 to 15 mg | of carbohydrate, |
| 0.5 to 6 mg | of one or more compounds of the anthocyan type and/or a compound of the flavonone type and/or pectin, |
| 0.5 to 6 mg | of vegetable wax and |
| 0.01 to 0.1 mg | of ethereal oils, |

are dissolved all at once or distributed in $C_2 - C_4$ alkanol to give 100 ml of solution.

2. Process according to claim 1, characterized in that

| | |
|---|---|
| 5 to 6 mg | of vitamins, trace elements, vegetable hormones, catalase enzymes with an oxidative action and/or inorganic salts |

are used as additional components.

3. Process according to claims 1 or 2, characterized in that

as the tanning agent pyrocatechol, tannin, gallic acid, digallic acid and/or pentadigalloyl-glucose,
as the carbohydrate glucose, fructose, rhamnose and/or xylose,
as a compound of the anthocyan type anthocyan and/or cyanidine,
as a compound of the flavonone type quercetin,
as essential oils 40 to 90% of geraniol, 5 to 15% of nerol, 10 to 50% of citronellol, eugenol, linalool or the corresponding aldehydes,
as vitamins A, $B_1$, $B_6$, $B_{12}$, C, K and/or P,
as trace elements manganese, magnesium, calcium, cobalt, iron and/or zinc,
as vegetable hormones auxin and kinetin in ng quantities,
as enzymes with an oxidative action peroxidase and/or catalase and
as inorganic salts phosphates and/or chlorides

are used.

4. Process according to claims 5, 6 or 7, characterized in that 70 to 100% ethanol is used as the solvent.


## Revendications pour les etats contractants: BE, CH/LI, DE, FR, GB, IT, NL

1. Préparation contenant du tannin pour le traitement des blessures de la surface de la peau, caractérisée en ce qu'elle contient dans 100 ml de solution d'alcanol en $C_2$ à $C_4$

| | |
|---|---|
| 5 – 20 mg | de tannin, |
| 5 – 15 mg | d'hydrate de carbone, |
| 0,5 – 6 mg | d'un ou plusieurs composés du type anthocyane et/ou d'un composé du type flavonone et/ou de pectine, |
| 0,5 – 6 mg | de cire végétale et |
| 0,01 – 0,1 mg | d'huiles éthérées. |

2. Préparation selon la revendication 1, caractérisée en ce qu'elle contient comme composants supplémentaires

| | |
|---|---|
| 5 – 6 mg | de vitamines, d'oligo-éléments, d'hormones végétales, de catalase, d'enzymes à action oxydante et/ou de sels inorganiques. |

3. Préparation selon les revendications 1 et 2, caractérisée en ce qu'elle contient

comme tannin la pyrocatéchine, le tannin, l'acide gallique, l'acide digallique et/ou le pentadigalloyl-glucose,
comme hydrate de carbone le glucose, le fructose, le rhamnose et/ou le xylose,
comme composé du type anthocyane l'anthocyane et/ou la cyanidine,
comme composé du type flavonone la quercétine,
comme huiles éthérées 40 – 90% de géraniol, 4 – 15% de nérol, 10 – 50% de citronellol, d'eugénol, de linalol ou selon les cas des aldéhydes correspondants,

13

**0 060 553**

comme vitamines les vitamines A, $B_1$, $B_6$, $B_{12}$, C, K et/ou P,
comme oligo-éléments le manganèse, le magnésium, le calcium, le cobalt, le fer et/ou le zinc,
comme hormones végétales l'auxine, la cinétine en quantités de l'ordre du nanogramme,
comme enzymes à action oxydante la peroxydase et/ou la catalase et
comme sels inorganiques les phosphates et/ou les chlorures.

4. Préparation selon les revendication 1, 2 et 3, caractérisée en ce qu'elle contient comme solvant de l'éthanol à 70—100%.

5. Procédé de préparation de préparations contenant du tannin pour le traitement de la surface de la peau, caractérisé en ce qu'on dissout en une ou plusieurs fois dans un alcanol en $C_2$ à $C_4$ pour donner 100 ml de solution

| | |
|---|---|
| 5—20 mg | de tannin, |
| 5—15 mg | d'hydrate de carbone |
| 0,5—6 mg | d'un ou plusieurs composés du type anthocyane et/ou d'un composé du type flavonone et/ou de pectine, |
| 0,5—6 mg | de cire végétale et |
| 0,01—0,1 mg | d'huiles éthérées. |

6. Procédé selon la revendication 5, caractérisé en ce qu'on utilise

5—6 mg     de vitamines, d'oligo-éléments, d'hormones végétales, de catalase, d'enzymes à action oxydante et/ou de sels inorganiques

comme composants additionnels.

7. Procédé selon les revendications 5 et 6, caractérisé en ce qu'on utilise

comme tannin la pyrocatéchine, le tannin, l'acide gallique, l'acide digallique et/ou le pentadigalloyl-glucose,
comme hydrate de carbone le glucose, le fructose, le rhamnose et/ou le xylose,
comme composé du type anthocyane l'anthocyane et/ou la cyanidine,
comme composé du type flavonone la quercétine,
comme huiles éthérées 40—90% de géraniol, 4—15% de nérol, 10—50% de citronellol, d'eugénol, de linalol ou selon les cas des aldéhydes correspondants,
comme vitamines les vitamines A, $B_1$, $B_6$, $B_{12}$, C, K et/ou P,
comme oligo-éléments le manganèse, le magnésium, le calcium, le cobalt, le fer et/ou le zinc,
comme hormones végétales l'auxine, la cinétine en quantités de l'ordre du nanogramme,
comme enzymes à action oxydante la peroxydase et/ou la catalase et
comme sels inorganiques les phosphates et/ou les chlorures.

8. Procédé selon les revendications 5, 6 et 7, caractérisé en ce qu'on utilise comme solvant de l'éthanol à 70—100%.

**Revendications pour l'Etat contractant: AT**

1. Procédé de préparation de préparations contenant du tannin pour le traitement de la surface de la peau, caractérisé en ce qu'on dissout en une ou plusieurs fois dans un alcanol en $C_2$ à $C_4$ pour donner 100 ml de solution

| | |
|---|---|
| 5—20 mg | de tannin, |
| 5—15 mg | d'hydrate de carbone, |
| 0,5—6 mg | d'un ou plusieurs composés du type anthocyane et/ou d'un composé du type flavonone et/ou de pectine, |
| 0,5—6 mg | de cire végétale et |
| 0,01—0,1 mg | d'huiles éthérées. |

2. Procédé selon la revendication 1, caractérisé en ce qu'on utilise comme composants supplémentaires

5—6 mg     de vitamines, d'oligo-éléments, d'hormones végétales, de catalase, d'enzymes à action oxydante et/ou de sels inorganiques.

3. Procédé selon les revendications 1 et 2, caractérisé en ce qu'on utilise

comme tannin la pyrocatécnine, le tannin, l'acide gallique, l'acide digallique et/ou le pentadigal-loyl-glucose,
comme hydrate de carbone le glucose, le fructose, le rhamnose et/ou le xylose,
comme composé du type anthocyane l'anthocyane et/ou la cyanidine,
comme composé du type flavonone la quercétine,
comme huiles éthérées 40—90% de géraniol, 4—15% de nérol, 10—50% de citronellol, d'eugénol, de linalol ou selon les cas des aldéhydes correspondants,
comme vitamines les vitamines A, $B_1$, $B_6$, $B_{12}$, C, K et/ou P,
comme oligo-éléments le manganèse, le magnésium, le calcium, le cobalt, le fer et/ou le zinc,
comme hormones végétales l'auxine, la cinétine en quantités de l'ordre du nanogramme,
comme enzyme à action oxydante la peroxydase et/ou la catalase et
comme sels inorganiques les phosphates et/ou les chlorures.

4. Procédé selon les revendications 1, 2 et 3, caractérisé en ce qu'on utilise comme solvant de l'éthanol à 70—100%.